Europäisches Patentamt

(19) European Patent Office

Office européen des brevets

(11) Veröffentlichungsnummer: **0 001 773**

**A1**

# EUROPÄISCHE PATENTANMELDUNG

(21) Anmeldenummer: 78101146.5

(22) Anmeldetag: 14.10.78

(51) Int. Cl.²: **C 07 G 7/00**
A 61 K 37/64, C 07 G 7/02

(30) Prioritat: 27.10.77 DE 2748294

(43) Veröffentlichungstag der Anmeldung:
16.05.79 Patentblatt 79 10

(84) Benannte Vertragsstaaten:
BE CH DE FR GB NL SE

(71) Anmelder: Bayer Aktiengesellschaft
Zentralbereich Patente,Marken und Lizenzen Bayerwerk
D-5090 Leverkusen 1(DE)

(72) Erfinder: Schnabel, Eugen, Dr.
Schimmelweg 6
D-5600 Wuppertal 11(DE)

(72) Erfinder: Reinhardt, Gerd, Dr.
Am Eckbusch 55a
D-5600 Wuppertal 1(DE)

(72) Erfinder: Schlumberger, Horst Dieter, Prof. Dr.
Schwartnerstrasse 5
D-5600 Wuppertal 2(DE)

(72) Erfinder: Opitz, Hans-Georg, Dr.
Steinberger Weg 52
D-5600 Wuppertal 17(DE)

(72) Erfinder: Truscheit, Ernst, Dr.
Horather Strasse 160
D-5600 Wuppertal 1(DE)

(72) Erfinder: Tschesche, Harald, Prof. Dr.
Im Strohsiek 24
D-4800 Bielefeld 1(DE)

(54) Neue Derivate des BPTI(basic pancreatic trypsin-inhibitor), Verfahren zu ihrer Herstellung sowie ihre Verwendung als Arzneimittel.

(57) Derivate des Trypsin-Kallikrein-Inhibitors aus Tierorganen, vorzugsweise aus Rinderorganen (BPTI, d.h. 'basic pancreativ trypsin-inhibitor' genannt) und ein Verfahren zu ihrer Herstellung durch Umsetzung von BPTI, oder BPTI-Derivaten mit freien Carboxylgruppen, mit Carbodiimiden und eine gegebenenfalls angeschlossene Desamidierung oder Azokupplung mit Diazoniumsalzen werden beschrieben, weiterhin die Verwendung dieser BPTI-Derivate als Arzneimittel, insbesondere als Arzneimittel, deren Wirkung auf einer proteaseinhibitorischen Funktion beruht.

EP 0 001 773 A1

BAYER AKTIENGESELLSCHAFT          5090 Leverkusen, Bayerwerk
Zentralbereich
Patente, Marken und Lizenzen      Lr/by/K

Neue Derivate des BPTI, Verfahren zu ihrer Herstellung
sowie ihre Verwendung als Arzneimittel

Die vorliegende Erfindung betrifft neue Derivate des Trypsin-Kallikrein-Inhibitors aus Tierorganen, vorzugsweise aus Rinderorganen (im folgenden BPTI, d.h. "basic panareatic trypsin-inhibitor" genannt), ein Verfahren zu ihrer Herstellung durch Umsetzung von BPTI oder BPTI-Derivaten mit freien Carboxylgruppen, mit Carbodiimiden und eine gegebenenfalls angeschlossene Desamidierung oder Azokupplung mit Diazoniumsalzen. Die Erfindung betrifft weiterhin die Verwendung dieser neuen BPTI-Derivate als Arzneimittel, insbesondere als Arzneimittel, deren Wirkung auf einer proteaseinhibitorischen Funktion beruht.

Es ist bereits bekannt geworden, daß BPTI /H.Kraut, E.K.Frey, E.Werle, Z.Physiol.Chem. 189, 97 (1930)/, auch als Kunitz-Inhibitor bezeichnet /M.Kunitz, H.H.Northrop, J.Gen.Physiol. 19, 991 (1936)/, eine Reihe von physiologisch bedeutsamen Enzymen, wie z.B. Kininogenine (Kininogenasen), Plasmin, Chymotrypsin und Trypsin hemmt (E.Werle in W.Brendel, G.L.Haberland: Neue Aspekte der Trasylol-Therapie 5, 9, F.K.Schattauer-Verlag Stuttgart-New York 1972; H.Fritz,

Le A 18 484-Ausland

0001773

- 2 -

H.Tschesche, L.J.Greene, E.Truscheit (Hrsg.): Proteinase Inhibitors (Bayer Symposium V), Proc. 2nd International Research Conference, Springer-Verlag Berlin-Heidelberg- New York 1974). BPTI wird als Aprotinin (Freiname) zur Therapie und Prophylaxe von Schockzuständen sowie zur Prophylaxe postoperativer und postraumatischer Komplikationen eingesetzt.

Es ist bekannt, daß Carboxylgruppen von Proteinen bei der Umsetzung mit Carbodiimiden mit den Aminogruppen der Proteine unter intra- und/oder intermolekularer Vernetzung reagieren können /J.C.Sheehan und J.J.Hlavka, J.Amer.Chem.Soc. $\underline{79}$, 4528 (1957)/. Ferner wurde unter gleichen Bedingungen die Bildung von Acylharnstoffderivaten der Proteine beobachtet. /J.P.Riehm und H.A.Scheraga, Biochemistry $\underline{5}$, 99 (1966)/.

Es wurde gefunden, daß man neue Derivate des BPTI, bei welchen ein Teil oder alle der Carboxylgruppen des nativen BPTI oder seiner freie Carboxylgruppen enthaltenden Derivate modifiziert sind, erhält, wenn man BPTI oder BPTI-Derivate mit freien Carboxylgruppen mit Carbodiimiden im wäßrigen oder wasserhaltigen Medium bei pH Werten zwischen 3 und 11 umsetzt, die entstandenen Reaktionsprodukte gegebenenfalls isoliert und gegebenenfalls anschließend desaminiert oder gegebenenfalls in ihre Azoderivate überführt. Weiterhin wurde gefunden, daß die erfindungsgemäßen neuen BPTI-Derivate stark ausgeprägte inhibitorische Wirkungen aufweisen.

Le A 18 484

0001773

- 3 -

Die Umsetzung von BPTI und den oben bezeichneten BPTI-Derivaten mit Carbodiimiden wird vorzugsweise bei pH-Werten zwischen 4,5 und 7,5 in wäßriger Lösung, die jedoch auch organische Lösungsmittel und/oder Salze und/oder Denaturierungsmittel enthalten kann, durchgeführt.

Die erfindungsgemäße Umsetzung erfolgt bevorzugt bei Temperaturen zwischen $-20^\circ$C und $+50^\circ$C, insbesondere zwischen $0^\circ$C und $30^\circ$C.

Pro Mol BPTI bzw. BPTI-Derivat werden 10-100 Mol, vorzugsweise 30 bis 60 Mol Carbodiimid eingesetzt.

Bei den Umsetzungen resultierende Gemische können gegebenenfalls nach an sich bekannten Verfahren, z.B. durch Elektrophorese, Gelfiltration oder Ionenaustauschchromatographie fraktioniert werden.

Als Carbodiimide können bei der erfindungsgemäßen Umsetzung zur Herstellung der erfindungsgemäßen Verbindungen eine Reihe von Carbodiimiden, vorzugsweise wasserlösliche organische Carbodiimide eingesetzt werden, insbesondere folgende Carbodiimide haben sich für die Durchführung des erfindungsgemäßen Verfahrens als besonders geeignet herausgestellt:
Dicyclohexylcarbodiimid,
N-Cyclohexyl-N'-/2-(4-morpholinyl)-äthyl7-carbodiimid-metho-toluol-4-sulfonat,
N-Äthyl-N'-(3-dimethylaminopropyl)-carbodiimid-hydrochlorid,

Le A 18 484

- 4 -

N-tert.-Butyl-N'-(3-dimethylaminopropyl)-carbodiimid,
N-Cyclohexyl-N'-(3-dimethylaminopropyl)-carbodiimid-hydrochlorid,
N-Isopropyl-N'-(3-dimethylaminopropyl)-carbodiimid-hydrochlorid,
N-Cyclohexyl-N'-(3-dimethylaminopropyl)-carbodiimid-metho-
toluol-4-sulfonat,
N,N'-Diisopropyl-carbodiimid,
N,N'-Di-para-tolyl-carbodiimid.

Beim erfindungsgemäßen Verfahren der Umsetzung von BPTI bzw. BPTI-Derivaten mit Carbodiimiden erfolgt die Aufarbeitung des Reaktionsproduktes vorzugsweise so, daß zunächst niedermolekulare Bestandteile des Reaktionsgemisches durch Filtrieren der Reaktionslösung über geeigneten Säulen (z.B. Sephadex ® G 10-Säule) vorzugsweise mit Säuren oder mit Salzlösungen bzw. mit wäßrigem Ammoniak als Elutionsmittel, abgetrennt werden. Insbesondere seien als Elutionsmittel Essigsäure und Ammoniumbicarbonatlösung genannt. Die Protein enthaltenden Eluate wurden vorzugsweise gefriergetrocknet und anschließend zum Abtrennen der höhermolekularen Anteile an einer geeigneten Säule chromatographiert.

Als geeignete Säulen kommen vorzugsweise folgende Säulen in Frage: quervernetzte Dextrane, Agarose-Säulen, Polyacrylamid-Säulen, es handelt sich in allen Fällen um Molekularsiebe und/oder Ionenaustauscher. Elutionsmittel sind wiederum vorzugsweise Säuren und Salzlösungen insbesondere Essigsäure, Pufferlösungen wie Ammoniumacetat und Natriumphosphat im Gemisch mit Kochsalz.

**BAD ORIGINAL**

Le A 18 484

0001773

BAD ORIGINAL

- 5 -

Weiterhin erhält man erfindungsgemäß neue Derivate des BPTI dadurch, daß man die durch Umsetzung von BPTI oder seinen Derivaten mit Carbodiimiden gewonnenen Derivate anschließend ganz oder teilweise desaminiert. Dazu werden die Derivate mit Nitrosylionen-liefernden Agentien wie Salzen, Estern oder gemischten Anhydriden der salpetrigen Säure in saurer Lösung gegebenenfalls in Gegenwart von Zusatzstoffen im heterogenen oder homogenen System umgesetzt.

Alternativ kann man die nachträgliche Desaminierung auch durch Umsetzen der Derivate mit geeigneten nicht kuppelnden Diazoniumverbindungen durchführen. Als nicht kuppelnde Diazoniumverbindungen sei das aus 5-Aminotetrazol durch Umsetzung mit Nitrit erhaltene Diazoniumsalz genannt.

Weiterhin erhält man erfindungsgemäß neue Derivate des BPTI dadurch, daß man die durch Umsetzung von BPTI oder seinen Derivaten, welche freie Carboxylgruppen enthalten, mit Carbodiimiden gewonnenen Derivate anschließend mit Diazoniumverbindungen in wäßriger Lösung, die gegebenenfalls auch organische Lösungsmittel wie beispielsweise Pyridin, Chinolin, Dimethylformamid, Dimethylsulfoxid oder Hexamethylphosphorsäuretriamid, und/oder Zusätze, wie Salze, beispielsweise Guanidinhydrochlorid oder Lithiumchlorid, und/oder Nichtelektrolyte, wie z.B. Harnstoff, enthalten kann, zu Azo-BPTI-Derivaten kuppelt.

Die Kupplungsreaktionen werden je nach der Reaktivität der Diazoniumverbindungen bei pH-Werten zwischen 1 und 12 vorzugsweise bei pH 5 bis 10 durchgeführt, wobei Lösungen des BPTI

Le A 18 484

0001773

- 6 -

oder von BPTI-Derivaten in Wasser oder in den oben aufgeführten Lösungsmitteln, die gegebenenfalls die oben angegebenen Zusatzstoffe enthalten, mit den Diazoniumsalzlösungen vereinigt werden. Der pH-Wert des Reaktionsgemisches wird durch Zugabe von Alkalimetall- bzw. Erdalkalimetall-Oxiden, -Hydroxiden, -Carbonaten, -Hydrogencarbonaten oder tert. Aminen, wie z.B. Triäthylamin, N-Methylmorpholin, Triäthanolamin oder Pyridin, eingestellt und konstant gehalten. Die Umsetzungen können aber auch in Pufferlösungen, insbesondere Phosphat- oder Boratpuffern durchgeführt werden. Die Umsetzungsgeschwindigkeiten können zweckmäßig über den pH-Wert des Reaktionsgemisches gesteuert werden.

Der Endpunkt der Umsetzung kann beispielsweise durch Tüpfelreaktion ermittelt werden, bei der eine Probe der Reaktionslösung mit 1-(2-Aminoäthylamino)-naphthalin als Kupplungspartner umgesetzt wird.

Während der Kupplungsreaktionen hält man die Temperaturen der Reaktionsgemische bei -15°C bis 30°C, vorzugsweise bei 0°C bis 10°C. Unter Umständen ist es zweckmäßig, die Kupplung zunächst bei Temperaturen zwischen 0°C und 4°C durchzuführen und anschliessend die Temperatur bis auf 25°C zu erhöhen. Überschüssige Diazoniumverbindung kann ggf. vor der Aufarbeitung des Reaktionsgemisches durch Zusetzen von z.B. von Phenol zur Reaktionslösung gebunden werden. Das molare Verhältnis von Diazoniumverbindungen zu eingesetztem BPTI-Derivat beträgt 1:1 bis 10:1, vorzugsweise 1:1 bis 5:1, wobei die Konzentrationen an BPTI-Derivaten in den zur Kupplung verwendeten Lösungen bei 1-30 %, vorzugsweise bei 5-15 % liegen.

Le A 18 484

Als Azokomponenten sind zur Darstellung der erfindungsgemäßen Azo-BPTI-Derivate ein- oder mehrkernige aromatische sowie heterocyclische Diazoniumverbindungen geeignet, die aus den entsprechenden Aminen dargestellt werden. Diese können noch einen oder mehrere Substituenten tragen. Insbesondere sind Derivate des Anilins geeignet, z.B. Alkylaniline, Alkoxyaniline, Halogenderivate des Anilins, Nitroaniline, Cyanoaniline, Trifluormethylaniline, Acetaminoaniline, Amino-acetophenone, Aminobenzolsulfonsäuren, Anilindisulfonsäuren, Aminobenzolarsonsäuren, Aminobenzoesäuren, Aminodiphenyläther; als mehrkernige Amine sind besonders ⍺- und ß-Naphthylamin sowie deren Derivate, z.B. Aminonaphthalinsulfonsäuren, geeignet.

Als Heterocyclen sind insbesondere Aminotriazol und Aminophenyltriazole, die auch substituiert sein können, brauchbar.

Die Diazotierung der bei der erfindungsgemäßen Azokupplung eingesetzten Amine wird nach den üblichen Verfahren /R.Pütter, in "Houben-Weyl, Methoden der organischen Chemie", Band 10/3, S. 1 ff., Hrsg. E.Müller unter Mitwirkung von O.Bayer, H.Meerwein und K.Ziegler, Georg Thieme Verlag, Stuttgart 1965/ durch Umsetzen mit Nitrosylionen-liefernden Verbindungen, insbesondere Natriumnitrit in mineralsaurer Lösung oder Suspension ggf. in Anwesenheit von organischen Lösungsmitteln, insbesondere von Dimethylformamid, Essigsäure oder Propionsäure, bei Temperaturen von -20°C bis 50°C, vorzugsweise -5°C bis 20°C, oder aber mit Nitrosylschwefelsäure bzw. Nitrosylchlorid in Essigsäure-Propionsäure-Gemischen unter Zusatz von Mineralsäure bei Temperaturen um vorzugsweise -20° bis 0° durchgeführt. Die Zeitdauer der Diazotierung ist sehr unterschiedlich; sie hängt ab von der Löslichkeit der Amine sowie deren Substitutionsmuster und schwankt zwischen wenigen Minuten und mehreren Stunden. Über-

Le A 18 484

- 8 -

schüssige Nitrosylionen werden nach der Diazotierung durch Umsetzen mit Amidosulfonsäure oder Harnstoff zerstört, um eine Desaminierung der BPTI-Derivate durch Nitrosylionen, wie sie beim Durchführen der Kupplungen im sauren Milieu möglich ist, zu verhindern.

Bei der Kupplung von BPTI-Derivaten mit den Diazoniumverbindungen werden Gemische verschiedener Komponenten erhalten. Die Zusammensetzung dieser Azo-BPTI-Derivat-Gemische kann durch die Wahl der Umsetzungsbedingungen, insbesondere über das Verhältnis von zur Kupplung eingesetztem BPTI-Derivat und Diazoniumverbindung, den pH-Wert der Reaktionsgemische, die Zeitdauer der Umsetzung sowie durch die obengenannten Zusätze zu den Reaktionslösungen gesteuert werden.

Wie bereits oben erwähnt, werden als Ausgangsprodukte zur Herstellung der erfindungsgemäßen carboxylmodifizierten Verbindungen BPTI und BPTI-Derivate mit freien Carboxylgruppen eingesetzt.

Als Ausgangsprodukte seien neben dem BPTI beispielhaft genannt:

1. Desamino-BPTI-Derivate, welche ganz oder teilweise desaminiert sein können und/oder an einem der beiden Tyrosinreste 10 oder 21 in ortho-Stellung zur phenolischen Hydroxylgruppen eine Nitrogruppe tragen; sie können auch am Tyrosinrest 10 und/oder 21 in ortho-Stellung zur phenolischen Hydroxylgruppe eine Aminogruppe enthalten. Man erhält sie aus BPTI oder Derivaten des BPTI nach einem der folgenden Verfahren:

- 9 -

a) Umsetzung derselben in saurer Lösung mit Nitrosylionen liefernden Agentien wie beispielsweise Alkali- oder Erdalkalinitriten,Estern der salpetrigen Säure oder gemischten Anhydriden der salpetrigen Säure, gegebenenfalls in Gegenwart von Zusatzstoffen, beispielsweise solchen, die die Konformation von Proteinen beeinflussen (z. B. organischen Lösungsmitteln, Detergentien oder Guanidiniumhydrochlorid) in heterogenem oder in homogenem System und Aufarbeitung der Reaktionsgemische nach an sich bekannten Methoden. Man erhält dabei Gemische von BPTI-Derivaten unterschiedlichen Desaminierungsgrades, von denen ein Teil auch an den Tyrosinresten 10 und/oder 21 nitriert worden ist. Diese Nitrierung läßt sich zurückdrängen, wenn man während der Desaminierungsreaktion unter Schutzgas oder Evakuierung arbeitet und/oder einen Akzeptor für Elektrophile, z. B. Phenol, zusetzt. Während der Aufarbeitung kann die Nitrierung durch Alkalisieren des Ansatzes verhindert werden.

b) Umsetzung derselben in saurer, neutraler oder schwach alkalischer Lösung mit Diazoniumverbindungen, die man durch Diazotierung von heterocyclischen Aminen, insbesondere von 5-Aminotetrazol, erhält und Aufarbeitung des Reaktionsgemisches nach an sich bekannten Methoden. Den Reaktionsansätzen können gegebenenfalls Zusatzstoffe, wie z.B. organische Lösungsmittel, Detergentien, Guanidiniumhydrochlorid beigegeben werden. Man erhält dabei Gemische von BPTI-Derivaten unterschiedlichen, im Vergleich zu Variante a) wesentlich geringeren, Desaminierungsgrades, aus deren Absorptionsspektren ersichtlich ist, daß weder deren Tyrosinreste zu Azo-Derivaten noch deren primäre Aminogruppen zu Triazenen umgewandelt worden sind.

0001773

- 10 -

2. Desamido-BPTI, hergestellt durch längeres (20-30 tägiges) Stehenlassen von nativer BPTI in wäßriger Säure (vorzugsweise 2n Salzsäure) bei Raumtemperatur.

3. Mononitro-BPTI erhalten nach B. Meloun et al. Europ. J. Biochem. 4, 112 (1968) durch direkte Nitrierung von BPTI mit Tetranitromethan oder erfindungsgemäß mit Salpetersäure.

4. Dinitro-BPTI ebenfalls erhalten nach B. Meloun et al. Europ. J. Biochem. 4, 112, (1968) oder erfindungsgemäß mit Salpetersäure.

5. Derivate des BPTI, die an den Tyrosinresten 10 und/oder 21 Aminogruppen tragen und aus den Derivaten gemäß 3) und 4) durch Reduktion nach an sich bekannten Methoden z.B. nach M. Sokolovsky et al. Biochem. Biophys. Res. Comm. 27, 20 (1967) erhalten werden können.

6. Guanidierter BPTI erhalten nach B. Kassell et al. Biochemistry 5, 3449 - 3453.

7. Acylderivate des BPTI erhalten aus BPTI durch Umsetzung mit aktivierten Carbonsäurederivaten sowie andere Derivate des BPTI, mit noch freien Carboxylgruppen.

Die erfindungsgemäßen BPTI-Derivate hemmen im Gegensatz zu BPTI Elastasen aus Pankreas und Granulozyten. Gegenüber anderen Proteasen wie Chymotrypsin, Pankreas-Kallikrein, Plasma-Kallikrein, Plasmin, Kathepsin G und Trypsin unterscheiden sich die Hemmspektren einzelner Derivate qualitativ und/oder quantitativ von dem des BPTI. Bei der Elastasehemmung handelt

Le A 18 484

- 11 -

es sich nicht um die von J.Pütter und G.Schmidt-Kastner,
Biochem.Biophys.Acta 127, 538 (1966) beschriebene Blockierung
des Substrats, die nur bei sehr hohen BPTI-Konzentrationen
erfolgt, sondern um eine Hemmung des Enzyms.

Diese Elastase-Hemmwirkung ist umso überraschender, als aus
dem derzeitigen Stand der Technik folgt /D.M.Blow, C.S.Wright,
D.Kukla, A.Rühlmann, W.Steigemann und R.Huber, J.Mol.Biol. 69,
137 (1972); R.Huber, D.Kukla, W.Steigemann, J.Deisenhofer und
A.Jones in H.Fritz, H.Tschesche, L.J.Greene und E.Truscheit
(Hrsg.), Proteinase Inhibitors (Bayer Symposium V) Proc.2nd
Intern.Res.Conference, S. 484, Springer-Verlag Berlin-Heidel-
berg-New York 1974/, daß BPTI substratartig mit seinem aktiven
Zentrum (Lysin-15-Rest) in die Spezifitätstasche der hemmbaren
Enzyme eingelagert wird und damit die Enzymaktivität blockiert.
Selbst wenn die Seitenkette des Lysin-15-Restes bei einigen
erfindungsgemäßen Derivaten desaminiert ist, ist nach dem derzeitigen Wissensstand nicht zu erwarten, daß sie in die relativ
kleine Spezifitätstasche der Elastasen (D.Shotton in H.Fritz und
H.Tschesche (Hrsg.), Proceedings of the International Research
Conference on Proteinase Inhibitors, S. 47, Walter de Gruyter,
Berlin-New York 1971) hineinpasst.

Die erfindungsgemässen BPTI-Derivate sind neu. Sie lassen
sich durch chemische, physikalisch-chemische, biochemische sowie
biologische Eigenschaften charakterisieren und gegenüber bekannter
Substanzen abgrenzen. Es wurden folgende Kriterien herangezogen:

BAD ORIGINAL

0001773

1.) Aminosäurezusammensetzung

Die Aminosäurezusammensetzung wurde nach S. Moore, D.H.Spackman, W.H.Stein, Anal.Chem. 30, 1185 (1958) bestimmt. Tabelle 1 gibt die Gehalte charakteristischer Aminosäuren für einige weitere Carboxyl-modifizierte BPTI-Derivate wieder. Weiterhin ist aus der Tabelle ersichtlich, wie sich der Gehalt dieser Aminosäuren nach einer anschliessenden Kupplungs- oder Desaminierungsreaktion verändert hat.

2.) Elektrophoretische Eigenschaften

Cellogelelektrophoresen: Für die Cellogelelektrophoresen wurden 2.5 cm breite Celluloseacetatstreifen der Fa. Serva unter den üblichen Bedingungen /J.Kohn, Cellulose Acetate Electrophoresis and Immuno-Diffusion Techniques in I.Smith, Chromatographic and Electrophoretic Techniques, Vol. II, W.Heinemann, Ltd., London and Interscience Publishers 56-90 (1960); Gelman Instrument Company (1968)/ verwendet. Der eingesetzte Pyridin-Acetat-Puffer pH 6.05 wurde durch Auffüllen eines Gemisches von 200 ml Pyridin und 20 ml Eisessig mit Wasser auf 2 Ltr. erhalten. Die Trennungen wurden bei 150 V mit einer Stromstärke von 0.8 mA / cm Streifenbreite durchgeführt, wobei man stets zum Vergleich BPTI als Standard mitlaufen ließ. Zum Anfärben der Streifen wurde eine Lösung von 5 g Amidoschwarz in einer Mischung von 900 ml Methanol und 100 ml Eisessig verwendet.

BAD ORIGINAL

Le A 18 484

<u>Disk-Elektrophoresen</u>: Die Disk-Elektrophoresen wurden in einem 15 %igen Trenngel mit 0.03 m Imidazol-Puffer pH 7.1 als Anodenpuffer und 0.03 m $\epsilon$-Aminocapronsäure, 0.01 m Imidazol-Puffer vom gleichen pH-Wert als Kathodenpuffer durchgeführt. Die Stromstärke betrug 2.5 mA pro Röhrchen; die Trennung wurde beendet, sobald der Markerfarbstoff (Methylgrün) vollständig durch das

*) Gelman Procedures, Techniques, and Apparatus for Electrophoresis,

<u>Le A 18 484</u>

Tabelle 1    Für einige Carboxyl-modifizierte BPTI-Derivate durch Aminosäureanalyse ermittelte Gehalte an charakteristischen Aminosäuren (Mol/Mol Derivat)[1]

| BPTI-Derivat nach Beispiel | hergestellt aus BPTI-Derivat nach Beispiel | Gly[2] | Ala[2] | Tyr | Lys | Arg |
|---|---|---|---|---|---|---|
| BPTI[3] | - | 6.09 | 6.00 | 3.89 | 4.08 | 6.01 |
| 1 | - | 5.78 | 5.91 | 3.90 | 4.01 | 5.95 |
| 4 | 1 | 5.64 | 5.45 | 3.87 | 0.32 | 5.09 |
| 5 | 1 | 5.21 | 5.82 | 1.71 | 3.77 | 5.97 |
| 6 | 1 | 5.22 | 5.48 | 2.51 | 2.95 | 5.13 |

0001773

- 15 -

1) Das Molekulargewicht der Derivate wurde wie für BPTI
   zu 6511 angenommen.

2) Der Gly- bzw. Ala-Gehalt dient als interner Standard.

3) Zum Vergleich wurden die Werte der Aminosäuren für BPTI
   in die Tabelle aufgenommen. Die theoretischen Werte betragen: Gly, Ala und Arg je 6, Tyr und Lys je 4.

**Le A 18 484**

Gel hindurchgewandert war. Nach der Trennung wurden die Gele in einer 0.1 %igen Lösung von Amidoschwarz in 7 %iger Essigsäure 30 Minuten lang gefärbt. Überschüssiger Farbstoff wurde anschließend mit 7 %iger Essigsäure wieder entfernt.

In den Beispielen ist als relative elektrophoretische Beweglichkeit jeweils der Quotient aus der Wanderungsstrecke des betreffenden BPTI-Derivates und der Wanderungsstrecke des BPTI angegeben. Das Vorzeichen gibt die Wanderungsrichtung an. Negatives Vorzeichen bedeutet also kathodische Beweglichkeit.

3.) Absorptions-Spektren

Zur weiteren Charakterisierung der erfindungsgemäßen BPTI-Derivate eignen sich besonders bei den Azo- und/oder Nitrogruppen enthaltenden Verbindungen die Absorptionsspektren. Charakteristische Daten sind für einige Derivate in den betreffenden Beispielen angegeben.

4.) Proteasen-Inhibitionsspektren

a) Elastase-Inhibition

∝) Pankreas-Elastase-Inhibition

Für die Hemmversuche mit den erfindungsgemässen BPTI-Derivaten wurde kristallisierte Pankreas-Elastase (Schwein) der Fa. Nutritional Biochemicals Corp. verwendet. Als Substrate wurden Elastin-Kongorot/M.A. Naughton und F.Sanger, Biochem.J. 78, 156 (1961)/, lösliches Elastin /S.Keller und I.Mandl, Biochem.Med. 5, 342 (1971)/ sowie Succinyl-L-alanyl-L-alanyl-L-alanin-p-nitroanilid /J.Bieth, B.Spiess und C.C. Wermuth, Biochem.Med. 11, 350 (1974)/ verwendet. Das synthetische Peptidsubstrat ermöglicht eine einfache collorimetrische Bestimmung der im Test eingesetzten Enzymaktivität. Der Elastase-Einsatz betrug im Elastin-Kongorot-Test ca. 1,1 nkat (vgl. Enzyme Nomenclature, Recommendations (1972) of the International Union of Pure and Applied Chemistry and the International Union of Biochemistry, 1973, Elsevier Amsterdam-New York, S. 26 - 27); mit löslichem Elastin als Substrat und ebenso mit Succinyl-L-alanyl-L-alanyl-L-alanin-p-nitroanilid wurden ca. 0.25 nkat Elastase pro Test-Ansatz eingesetzt. Zur quantitativen Bestimmung der Elastase-Hemmung wurden die oben angegebenen Enzym-Mengen mit Inhibitorlösungen definierter Konzentration versetzt und die Substratlösung zugegeben. Um maximale Komplexbildung sicherzustellen, wurden in einigen Fällen Enzym und Inhibitor vor Zugabe des Substrats 15 Minuten vorinkubiert.

Die Hydrolyse des Substrats wurde beim Elastin-Kongorot-Test durch Messung der Extinktion bei 492 nm der nach einer definierten Zeit gebildeten löslichen Spaltprodukte bestimmt. Beim Test mit löslichem Elastin wurde die Hydrolyse-Rate durch photometrische Bestimmung der nach einer definierten Zeit in Lösung gegangenen mit Ninhydrin färbbaren Spaltprodukte ermittelt. Bei Verwendung von Succinyl-L-alanyl-L-alanyl-L-alanin-p-nitroanilid als Substrat wurde die Hydrolyse durch kontinuierliche Messung der Extinktion des freigesetzten p-Nitroanilins bei 410 nm bestimmt. Die Hemmwerte (ausgedrückt

BAD ORIGINAL

- 18 -

in % Hemmung) wurden ermittelt, indem man die nach Inhibitorzusatz gemessene Elastase -Restaktivität von der Aktivität
der Enzymkontrolle subtrahierte. Die Hemmwerte einiger
erfindungsgemäßer BPTI-Derivate sind in Tabelle 2
zusammengestellt.

### a) Granulozyten-Elastase-Inhibition

Das für die Hemmteste verwendete Isoenzymgemisch wurde nach
K.Ohlsson und I.Olsson /Europ.J.Biochem. 42, 519 (1974)/
aus Humangranulozyten gewonnen. Als Substrat ist besonders
Succinyl-L-alanyl-L-alanyl-L-alanin-p-nitroanilid /J.Bieth,
B.Spiess und C.G.Wermuth, Biochem.Med. 11, 350 (1974)/
geeignet. Die Hemmwerte einiger BPTI-Derivate sind in
Tabelle 2 aufgenommen; der Enzym-Einsatz betrug 0.25 nkat
pro Test. Die Hemmwerte sind Absolutwerte und nicht auf
BPTI bezogen. Sie wurden ermittelt, wie für Pankreas-Elastase
angegeben.

### b) Chymotrypsin-Inhibition

Bei der Bestimmung der Aktivität von Chymotrypsin diente
Succinyl-L-phenylalanin- ß-naphthylester in einem 0.25 m
Trispuffer pH 7.2, der 5 m M an Calciumchlorid war, als
Substrat. Das bei der Spaltung gebildete ß-Naphthol wurde
photometrisch bei 329 nm bestimmt.

2 mg kristallisiertes α-Chymotrypsin wurden in 5 ml 0.001 -Salz
säure, die 5 m M an Calciumchlorid war, gelöst und 0.5 ml
dieser Lösung mit 0.25 m Trispuffer pH 7.2 (s.oben) auf
10 ml aufgefüllt. 50 µl der so erhaltenen Enzymlösung fügte
man zu 2.35 ml des 0.25 m Trispuffers und versetzte diese
Lösung für die Enzymkontrolle mit 100 µl Wasser, für den
Inhibitionstest jedoch mit 100 µl einer BPTI-Derivatlösung mit
bekanntem Gehalt. Nach gutem Durchmischen und einer Äquili-

BAD ORIGINAL

- 19 -

brierphase von 15 Minuten oder ggf. auch ohne Vorinkubation setzte man 25 µl der Substratlösung zu. Die Substratlösung wurde hergestellt durch Lösen von 39.1 mg Succinyl-L-phenylalanin-ß-naphthylester in 1 ml Dimethylsulfoxid. Die durch den Inhibitor bewirkte prozentuale Hemmung errechnet sich nach der folgenden Gleichung:

$$\frac{1 - \triangle OD\ Hemmansatz}{\triangle OD\ Kontrolle} \cdot 100 = \%\ Hemmung$$

wobei die optische Dichte um den durch Spontanhydrolyse des Naphthylesters bedingten ß-Naphthol-Anteil korrigiert wurde.

Die in der Tabelle 2 für einige BPTI-Derivate angegebenen Hemmwerte sind auf die durch die gleiche Menge BPTI bewirkte Hemmung (= 100 %) bezogen.

c) Cathepsin G - Inhibition

Das Enzym wurde nach einem kombinierten Verfahren nach J.R.Baugh und J.Travis /Biochemistry 15, 836 (1976)/ und nach W.Schmidt und K.Havemann /Z.Physiol.Chem. 355, 1077 (1974), isoliert. Die Bestimmung der Aktivität erfolgte, wie für Chymotrypsin beschrieben, mit Succinyl-L-phenylalanin-ß-naphthylester als Substrat in einem 0.25 m Trispuffer pH 7.2, der 0.01 % Brij (R) und 0.005 Mol/1 Magnesiumchlorid enthielt. Der Enzym-Einsatz betrug 0.25 nkat pro Test. Die Berechnung der prozentualen Hemmung wurde, wie unter b) beschrieben, durchge-führt.

BAD ORIGINAL

Le A 18 484

**d)** <u>Kininogenasen-Inhibition</u>

<u>∝) Plasma-Kininogenasen-Inhibition</u>

Die Kininogenasen-Aktivität wurde nach der Deutschen Offenlegungsschrift 25 27 932 vom 22.1.76, Erfinder L.G.Svendsen, Pentapharm AG., Basel, mit Benzoyl-L-prolyl-L-phenylalanyl-L-arginin-p-nitroanilid als Substrat bestimmt mit einem Einsatz von 0.1 nkat Enzym je Test. Das Gemisch der Isoenzyme wurde nach C.Sampaio et al. /Arch.Biochem.Biophys. 165, 133 (1974)/ durch Affinitätschromatographie aus der Human-Flasmafraktion Cohn IV-1 isoliert. Die in Tabelle 2 für einige BPTI-Derivate aufgeführten Hemmwerte wurden, wie unter Chymotrypsin-Inhibition angegeben, ermittelt.

<u>ß) Pankreas-Kininogenase-Inhibition</u>

Das Testenzym wurde nach C.Kutzbach und G.Schmidt-Kastner /Z.Physiol.Chem. 353, 1099 (1972)/ erhalten, und die Aktivitätsbestimmungen wurden nach denselben Autoren (s.o.) durchgeführt. Der Enzym-Einsatz betrug 10 nkat pro Test. Die in Tabelle 2 für einige BPTI-Derivate aufgeführten Hemmwerte wurden, wie unter Chymotrypsin-Inhibition angegeben, ermittelt.

**e)** <u>Plasmin-Inhibition</u>

Die enzymatische Aktivität von Plasmin wurde mit Azokasein bestimmt. Als Enzym wurde mit Urokinase aktiviertes Plasminoger benutzt, das mittels Affinitätschromatographie /D.G.Deutsch, E.T.Merz; J.Med. 3, 224 (1972)/ aus Humanplasma gewonnen wurde.

<u>Le A 18 484</u>

- 21 -

Die Herstellung des Substrates und das Prinzip des Testes sind in der Arbeit von P.M.Starkey, A.J.Barrett; Biochem.J. 155, 255 (1976) beschrieben. Man bereitet für jede Bestimmung zwei Ansätze A und B mit je 0.6 CU Plasmin, gelöst in 1.0 ml Puffer (0.1 m Tris-(hydroxymethyl)-aminomethan, 0.05 M NaCl, eingestellt auf pH 7.2 mit HCl). Beide Ansätze erhalten je 1.25 μg BPTI bzw. BPTI-Derivat, gelöst in 1.25 ml Puffer (s.o.); zum Vergleich dient je ein Ansatz A und B mit 1,25 ml Puffer anstelle des Inhibitors. Man inkubiert alle Ansätze 10 Min. bei 37°C und gibt darauf je 0,45 ml einer 2 %igen Azokaseinlösung (in Puffer, s.o.) zu. Serie A versetzt man direkt danach mit 0,3 ml 30 %iger Trichloressigsäurelösung (TCA); Serie B wird 60 Min. bei 37°C inkubiert und darauf mit 0,3 ml TCA versetzt. Die Ansätze werden nach ca. 20 Min. zentrifugiert; die Extinktionen der Überstände werden photometrisch bei 340 nm in einer 1 cm-Küvette bestimmt. Als Maß der enzymatischen Aktivität gilt die Extinktionsdifferenz der parallelen Ansätze A und B ( $\Delta E$ ).

Bezeichnet man die Extinktionsdifferenz, die von Plasmin allein hervorgerufen wird, mit $\Delta E_{100}$ und die in Gegenwart von Inhibitoren gemessene Extinktionsdifferenz mit $\Delta E$, so ergibt sich die Hemmung des Plasmins durch die Inhibitoren unter den angegebenen Bedingungen durch den folgenden Ausdruck

$$\frac{\Delta E_{100} - \Delta E}{\Delta E_{100}} \cdot 100 \%$$

BAD ORIGINAL

- 22 -

Die in Tabelle 2 für einige BPTI-Derivate aufgeführten Hemmwerte (ausgedrückt in %) werden auf die durch die gleiche
Menge BPTI bewirkte Hemmung (≙ 100 %) bezogen.

f) Trypsin-Inhibition

Die Bestimmung von Trypsin wurde mit Benzoyl-L-arginin-
äthylester-hydrochlorid als Substrat im pH-Stat-Verfahren
nach R.Ruyssen (Symposium on Pharmaceutical Enzymes and their
Assay, Universitaire Pers, Ghent, Belgium 1969, S. 110) durchgeführt. Die in Tabelle 2 für einige BPTI-Derivate aufgeführte
Hemmwerte wurden, wie unter Chymotrypsin-Inhibition angegeben,
ermittelt.

BAD ORIGINAL

Le A 18 484

**Tabelle 2**    Hemmung einiger Serinproteasen durch BPTI-Derivate

| BPTI-Derivate aus Beispiel | Chymotrypsin | Cathepsin G | Elastase (Pankr.) | Enzym Elastase (Gran.) | Kallikrein (Pankr.) | Kallikrein (Plasm.) | Plasmin | Trypsin |
|---|---|---|---|---|---|---|---|---|
| BPTI | 100 | 100 | O | O | 100 | 100 | 100 | 100 |
| 1 | 148 | 65 | O | 21 | 61 | 92 | 56 | 92 |
| 4 | 83 | 151 | 93 | 43 | 5 | 7 | O | 28 |
| 6 | 88 | O | 19 | 29 | 14 | 55 | 45 | 46 |

- 23 -

- 24 -

Für Chymotrypsin, Cathepsin G, die Kallikreine, Plasma und Trypsin sind die angegebenen Hemmwerte bezogen auf die durch die gleiche Menge BPTI bewirkte Hemmung (100 %). Die BPTI-Menge im Hemmtest wurde so gewählt, daß die Absolutwerte der Hemmung 50 ± 10 % betrug, außer beim Cathepsin G, wo der Absolutwert bei 20 % lag.

Bei den Elastasen enthält die Tabelle Absolutwerte. Je Test wurden 25 µg BPTI-Derivat eingesetzt.

Le A 18 464

0001773

- 25 -

<u>Versuchsanordnung zum Nachweis entzündungshemmender Wirkung</u>
<u>bei der Ratte</u>

a) <u>Kaolin-induzierte Entzündungsreaktion</u>

Die Entzündungsreaktion wurde durch intraplantare Injektion von 0,1 ml einer 10 %igen Kaolinsuspension in eine Hinterpfote von 130-160 g schweren Wistarratten induziert. Die für die Behandlung der Entzündungsreaktion verwendeten erfindungsgemässen BPTI-Derivate sowie BPTI wurden in 0,9 %iger Natriumchloridlösung in einer Konzentration von 10-20 mg/ml gelöst. Die Behandlung der Versuchstiere erfolgte durch intraperitoneale, intramuskuläre, subkutane oder intravenöse Injektion von 0,5 - 1,0 ml Lösung von BPTI-Derivaten und zum Vergleich BPTI entweder <u>prophylaktisch</u>, d.h. <u>vor</u> Setzen der Entzündungsnoxe, oder <u>therapeutisch</u>, d.h. <u>nach</u> Setzen der Entzündungsnoxe. Die Schwellung der entzündeten Pfote, die ein Maß für die Schwere der Entzündungsreaktion ist, wurde mit dem Antiphlogmeter nach Kemper /F.Kemper und G.Ameln, Z.ges.exp.Med. <u>131</u>, 407-411 (1959)/ zeitlich verfolgt.

Zur Ermittlung der Dosis-Wirkungsbeziehungen wurde der 4 Stunden nach Setzen der Entzündungsnoxe gemessene Wert verwendet.

Der Wirkungsvergleich der BPTI-Derivate zeigt, daß sie dem BPTI in ihrer entzündungshemmenden Wirkung überlegen sind.

BAD ORIGINAL

Le A 18 484

- 26 -

b) Aerosil-induzierte Entzündungsreaktion

Die Entzündungsreaktion wurde durch intraplantare Injektion von 0,1 ml einer 2 %igen Aerosilsuspension in eine Hinterpfote von 130-160 g schweren Wistarratten induziert. Die für die Behandlung der Entzündungsreaktion verwendeten erfindungsgemäßen BPTI-Derivate bzw. BPTI wurden in 0,9 %iger Natriumchloridlösung in einer Konzentration von 10-20 mg/ml gelöst. Die Behandlung der Versuchstiere erfolgt durch intraperitoneale, subkutane oder intravenöse Injektion von 0,5-1,0 ml Lösung von BPTI-Derivaten und zum Vergleich BPTI 15 Stunden nach Setzen der Entzündungsnoxe. Die Schwellung der entzündeten Pfote, die ein Maß für die Schwere der Entzündungsreaktion ist, wurde mit dem Antiphlogmeter nach Kemper zeitlich verfolgt. Zur Ermittlung der Dosis-Wirkungsbeziehungen wurde der 21-Stundenwert nach Entzündungsinduktion (= 6 Stunden nach Injektion der erfindungsgemäßen BPTI-Derivate bzw. des BPTI) ermittelt.

Das Ergebnis der Therapieversuche mit den BPTI-Derivaten nach den Beispielen zeigt die Wirksamkeit der verwendeten BPTI-Derivate in diesem experimentellen Modell, in dem BPTI in gleicher Dosierung die Entzündungsreaktion nicht hemmt.

Le A 18 484

Die erfindungsgemäßen BPTI-Derivate zeigen also höchst wertvolle therapeutische Eigenschaften. Von besonderem Vorteil sind ihre inhibitorischen Wirkungen auf die Elastasen aus Pankreas und Granulozyten, die neue therapeutische Einsatzmöglichkeiten eröffnen.

Pankreas-Elastase spielt eine wichtige Rolle bei der Pankreatitis /M.C.Geokas, H.Rinderknecht, V.Swanson, B.P.Vitron und B.J. Haverback, Clin.Res. 16, 285 (1968)/; Serum-Elastase bei der Atherosklerose /U.Butturini und M.Langen, Klin.Wochenschr. 40, 472 (1962)/ und Granulozyten-Elastase bei akuten und chronischen Entzündungen mit Bindegewebsschädigung /A.Janoff, Amer.J.Pathol. 68, 579 (1972)/, bei Gefäßwandschädigungen /A.Janoff und J.D. Zeligs, Science 161, 702 (1968)/, bei nekrotisierenden Erkrankungen und Degeneration von Lungengewebe, z.B. beim Emphysem / G.M.Turino, R.M.Senior, B.D.Garg, S.Keller, M.M.Levi und I.Mandl, Science 165, 709 (1969); H.E.Evans, M.M.Levi und I.Mandl, Amer.Rev.Respir.Dis. 101, 359 (1970) sowie A.Janoff, R.A.Sandhaus, V.D.Hospelhorn und R.Rosenberg, Proc.Soc.Exptl.Biol.Med. 140, 516 (1972)/. Ebenso wichtig ist die Rolle von lysosomalen Enzymen und insbesondere der Granulozyten-Elastase bei immunologisch bedingten Entzündungsreaktionen / M.Koono, M.Muto und H.Hayashi, Tohoku J.Exptl.Med. 94, 231 (1968) / , z.B. der rheumatoiden Arthritis / G.Weissmann und J.Spilberg, Arthritis Rheumat. 11, 162 (1968) /.

Es wurde gefunden, daß die erfindungsgemäßen BPTI-Derivate in Modellen der akuten Entzündungsreaktion dem BPTI überlegen sind, da mit ihnen nicht nur in deutlich geringeren Dosierungen die gleiche Wirkung wie mit BPTI erreicht wird, sondern die Entzündungsreaktion auch dann signifikant gehemmt wird, wenn sie mehrere Stunden nach Setzen der Entzündungsnoxe verabreicht werden. Eine solche therapeutische Wirkung ist mit dem BPTI im Kaolin- und Aerosilmodell bei einmaliger Gabe nicht zu erreichen.

Diese im Vergleich zu BPTI veränderte Wirkung und Wirksamkeit der erfindungsgemäßen BPTI-Derivate sind auf das veränderte Hemmspektrum und andere veränderte Eigenschaften, wie Le A 18 484

- 28 -

grössere Verweildauer und Wirkungszeit im Körper der Versuchstiere, zurückzuführen. Die erfindungsgemäßen BPTI-Derivate
sind deshalb biologisch deutlich vom BPTI abzugrenzen.

Die neuen erfindungemäßen BPTI-Derivate des BPTI können
aufgrund ih.er biologischen Wirksamkeit insbesondere zur Behandlung folgender Krankheiten bzw. Krankheitserscheinungen
eingesetzt werden:

1. Verschiedene Formen des Schocks, posttraumatischer und
   postoperativer Komplikationen
2. Störungen der Blutgerinnung
3. Akute und chronische Entzündungsreaktionen, insbesondere
   zur Therapie und Prophylaxe von Organschädigungen, wie
   beispielsweise Pankreatitis und Strahlen-induzierte
   Enteritis
4. Immunkomplex-bedingte Entzündungsreaktionen, wie Immun-
   Vasculitis, Glomerulonephritis und Arthritiden
5. Kollagenosen, insbesondere rheumatoide Arthritis
6. durch Stoffwechsel-bedingte Ablagerungen verursachte
   Arthritiden (z.B. Gicht)
7. Degeneration der elastischen Bestandteile der Bindegewebsbestandteile von Organen wie bei der Atherosklerose und
   Lungenemphysem.

Die neuen Wirkstoffe können in bekannter Weise (analog BPTI)
in die üblichen Formulierungen überführt werden.

Folgende Formulierungen sind dabei bevorzugt zu nennen:

1. Lösungen für parenterale Anwendung zur i.v.-, i.m.-,
   s.c.-Injektion, zur intraartikulären- und intra-
   tumoralen-Injektion
2. Lösungen für intravenöse Dauerinfusion
3. Lösungen zur Anwendung als Aerosole zur Inhalation
4. Lösungen, Emulsionen, Salben, Pasten, Cremes, Lotions,
   Puder zur äußerlichen lokalen Anwendung
5. Kombination verschiedener Hemmstoffe, deren Wirkungsspektrum sich gegenseitig ergänzt.

Le A 18 484

Die Konzentrationen der neuen Wirkstoffe in den erfindungsgemäßen Formulierungen bewegen sich in den Grenzen 0,01 bis
100 mg/ml Lösung, vorzugsweise zwischen 0,1 und 10 mg/ml
Lösung.

Die neuen Wirkstoffe können in üblicher Weise angewendet
werden, insbesondere sind folgende Anwendungsmethoden als
bevorzugt zu nennen:

1. parenteral, intravenös, intramuskulär, subkutan, intraartikulär, intratumoral
2. lokal; als Aerosol
3. oral, evtl. in Magensaft-resistenten Anwendungsformen
   und/oder Dünndarm-löslich.

Als Dosierungsbereich kann für die neuen erfindungsgemäßen
Verbindungen angegeben werden:

0,1 - 40 mg Wirkstoff / kg Körpergewicht, vorzugsweise
1 bis 25 mg Wirkstoff / kg Körpergewicht, die Dosierung ist
dabei vor allem abhängig von der zu behandelnden Spezies
sowie von der Applikationsart.

Die erfindungsgemäßen, neuen Wirkstoffe können bei Mensch
und Tier eingesetzt werden.

Die im vorliegenden Anmeldungstext verwendeten Warenzeichen
besitzen folgende Bedeutung:

Sephadex [R] G 10 = Quervernetztes Dextan mit einer Molgewichtsausschlußgrenze von 700

Sephadex [R] G 50 = Quervernetztes Dextan mit einer Molgewichtsausschlußgrenze von 30 000

BAD ORIGINAL

- 30 -

Beispiel 1

Zu einer Lösung von 1.3 g BPTI (200 μ Mol) in 130 ml Wasser fügte man unter Rühren 2.5 g N-Äthyl-N'-(3-dimethylaminopropyl)-carbodiimid-hydrochlorid (12 m Mol) und stellte durch Zugabe von n-Salzsäure den pH-Wert der Lösung auf 4,75 ein. Man hielt diesen pH-Wert mit Hilfe eines Autotitrators konstant. Nach 4 Std. wurde das Volumen der Reaktionslösung durch Einengen i.Vak. auf 15 ml reduziert und der Ansatz zum Abtrennen der niedrigmolekularen Reaktionsprodukte mit 0,1 m Essigsäure als Elutionsmittel über eine 100 x 2,5 cm quervernetzte Polydextrangel (Sephadex [R] G 10)-Säule filtriert. Die Protein enthaltenden Eluate wurden nach dem Gefriertrocknen zum Abtrennen der höhermolekularen Anteile (235 mg - 18 % -) mit 0,1 m Essigsäure an einer quervernetzten Polydextrangelsäule (Sephadex [R] G 50) chromatographiert. Man erhielt 1080 mg (82 %) farblose Substanz. In der Cellogelelektrophorese erwies sich die Substanz als stärker basisch als BPTI. Relative elektrophoretische Beweglichkeit - 1,78 (Cellogel) bzw. - 1,20 (Diskelektrophorese);

$/ \alpha / \frac{22}{578} = - 105,5^{\circ}$ (c = 0,5, in Wasser).

Beispiel 2 .

1.3 g BPTI (200 μ Mol) und 1.9 g N-tert.Butyl-N'-(3-dimethylaminopropyl)-carbodiimid wurden in 15 ml Wasser, wie in Beispiel 1 beschrieben, bei einem pH-Wert von 4.75 umgesetzt und der Ansatz entsprechend aufgearbeitet. Man isoliert nach der Chromatographie an quervernetztem Polydextrangel (Sephadex [R] G 50) 145 mg Polymere (11 %) und 1150 mg monomere Substanz (88 %).

Le A 18 484

- 31 -

Relative elektrophoretische Beweglichkeit: - 1,66 (Cellogel)
bzw. - 1,18 (Diskelektrophorese); Proteingehalt: 76 %;

$/\alpha/ \frac{22}{578} = -84.4^{\circ}$ (C = 0,5, in Wasser).


Beispiel 3

1.3 g BPTI (200 µ Mol) und 4.3 g N-Cyclohexyl-N'-/2-(morpholinyl)
äthyl/-carbodiimid-metho-p-toluolsulfonat (10 m Mol) wurden in
60 ml Wasser gelöst und die Lösung, wie in Beispiel 1 beschrieben
bei einem pH-Wert von 4.75 gerührt und aufgearbeitet. Durch
Chromatographie an Sephadex[R] G 50 mit 0,1 m Essigsäure als
Elutionsmittel erhielt man neben 98 mg polymeren Anteilen (8 %)
940 mg (72 %) farblose monomere Substanz.

Relative elektrophoretische Beweglichkeit: - 1.75 (Cellogel) bzw,
1.06 (Diskelektrophorese).

$/\alpha/ \frac{22}{578} = -99.5^{\circ}$ (c = 0.5, in Wasser).



Beispiel 4

250 mg des nach Beispiel 1 dargestellten BPTI-Derivates wurden
in 100 ml sauerstofffreier 0.25 m Essigsäure gelöst. In diese
Lösung wurden unter Rühren und Aufrechterhalten einer Stickstoffatmosphäre bei 4°C innerhalb von 30 Min. 25 ml 2 m Natriumnitritlösung zufliessen gelassen. Das Reaktionsgemisch wurde weitere
24 Std. bei 4°C gerührt. Dann stellte man den pH-Wert der Lösung
mit konz. wässrigem Ammoniak auf 8.0 ein und engte das Volumen

Le A 18 484

0001773

- 32 -

der Lösung mit Hilfe einer Ultrafiltrationszelle auf 10 ml ein. Man entsalzte die Lösung durch Filtration über quervernetztem Polydextrangel Sephadex[R] G 10 mit 0,1 m Ammoniak als Elutionsmittel. Beim Lyophilisieren des Protein enthaltenden Eluates wurden 146 mg (58 %) farblose Substanz erhalten.

Relative elektrophoretische Beweglichkeit: - 0,16 (Cellogel) bzw. - 0,62 und - 0,70 (Doppelbande; Diskelektrophorese);

Beispiel 5

Zur Lösung von 250 mg nach Beispiel 1 erhaltenem BPTI-Derivat wurden unter Rühren bei 4°C 100 ul Citraconsäureanhydrid (1.1 mMol) zufließen gelassen, wobei der pH-Wert der Lösung durch Zugabe von 8 n Natronlauge bei 8,0 gehalten wurde. Das Reaktionsgemisch wurde anschließend weitere 30 Minuten bei 4°C gerührt und mit einer aus 25 mg 3,5-Dichloranilin hergestellten Lösung der entsprechenden Diazoniumverbindung vereinigt und bei pH 8,0 gekuppelt. Herstellung der Diazoniumverbindung und Kupplung erfolgen wie nachstehend beschrieben:

Die Lösung von 25 mg 3,5-Dichloranilin (155 uMol) in 0,5 ml einer 1 + 1 Mischung von Essigsäure und 80 %iger Phosphorsäure wurde bei 4°C unter Rühren mit einer Lösung von 25 mg Natriumnitrit (180 uMol) in 50 ul Wasser versetzt. Nach 10 Min. fügte man 15 mg Harnstoff hinzu und vereinigte die Reaktionslösung nach weiteren 10 Minuten mit einer eiskalten Lösung von 250 mg des nach Beispiel 1 dargestellten BPTI-Derivates in 4 ml 0,1 m Natriumtetraboratlösung, pH 9,2. Man stellte den pH-Wert der Reaktionsmischung durch Zugabe von eiskalter 8 n

Le A 18 484

- 33 -

Natronlauge auf 8,0 ein, wobei ein Niederschlag entstand. Nach 1-stündigem Rühren bei 4$^{o}$C wurden 25 mg Phenol zu der Mischung gegeben und diese schließlich mit 8 ml Eisessig verdünnt. Die so erhaltene klare, intensiv rote Lösung wurde zur Abspaltung der Citraconylgruppen zusätzlich mit konzentrierter Salzsäure versetzt, bis das Reaktionsgemisch einen pH-Wert von 3,5 erreicht hatte. Nach 24-stündigem Stehen bei 20$^{o}$C wurde die Lösung zum Abtrennen der niedermolekularen Komponenten mit 50 %iger Essigsäure über quervernetztes Polydextrangel (Sephadex$^{(R)}$ G 10) filtriert. Polymere Reaktionsprodukte wurden durch Chromatographie an quervernetztem Polydextrangel (Sephadex$^{(R)}$ G 50) abgetrennt. Beim Gefriertrocknen der Monomeren-Fraktion wurden 178 mg braunes Azoderivat (71 %) erhalten.

Relative elektrophoretische Beweglichkeit: - 0,87 (Cellogel) bzw. - 0,95 (Diskelektrophorese);

$E \, {}^{1 \, cm}_{328} = 14,3$ (bei 6,5 g/l, 0,1 m Tris-Puffer, pH 7,5);

Aminosäureanalyse: vgl. Tabelle 1.


Beispiel 6

Aus 250 mg (78 uMol) nach Beispiel 1 dargestelltem BPTI-Derivat wurde, wie für Beispiel 5 angegebenen, die Citraconylverbindung hergestellt und diese mit dem aus 28 mg 3-Amino-1,2,4-triazol (330 uMol) und 25 mg Natriumnitrit (365 uMol) in der üblichen Weise hergestellten Diazoniumsalz bei pH 8.5 und 4$^{o}$C in das entsprechende Azoderivat übergeführt. Nach dem Abspalten der Citraconylreste und Abtrennen der niedermolekularen Begleit-

Le A 18 484

0001773

- 34 -

stoffe und Salze durch Filtration über quervernetztem Polydextrangel (Sephadex$^{(R)}$ G 10) mit 50 %iger Essigsäure wurden
198 mg (79 %) braune Azoverbindung erhalten.

Relative elektrophoretische Beweglichkeit: 0,0 und - 0,78
(Cellogel) bzw. - 0,92, - 0,82, - 0,68 und - 0,52 (Diskelektrophorese);

$$E \ ^{1 \ cm}_{328} \ = 23,5 \ (\text{bei } 6,5 \ g/l, \ 0,1 \ m \ \text{Tris-Puffer, pH } 7,5);$$

Aminosäureanalyse: vgl. Tabelle 1.

Le A 18 484

BAD ORIGINAL

- 35 -

Patentansprüche

1. Derivate des BPTI, bei welchem ein Teil oder alle der Carboxylgruppen des nativen BPTI oder seiner durch Desamidierung erhaltenen Desamidoderivate modifiziert sind, erhältlich durch Umsetzung von BPTI oder BPTI-Derivaten welche freie Carboxylgruppen enthalten mit Carbodiimiden in wäßrigem oder wasserhaltigem Medium bei pH-Werten zwischen 3 und 11, gegebenenfalls Isolierung der entstandenen Reaktionsprodukte und/oder gegebenenfalls anschließende Desaminierung und/oder gegebenenfalls anschließende Überführung in Azoderivate.

2. Derivate des BPTI gemäß Anspruch 1, dadurch gekennzeichnet, daß sie durch Umsetzung von BPTI oder BPTI-Derivaten welche freie Carboxylgruppen enthalten, mit Carbodiimiden und anschließende Isolierung der Reaktionsprodukte erhalten werden.

3. Derivate des BPTI gemäß Anspruch 1, dadurch gekennzeichnet, daß sie durch Umsetzung mit Nitrosylionen-liefernden Agenzien oder mit nicht kuppelnden Diazoniumverbindungen desaminiert worden sind.

4. Derivate des BPTI gemäß Anspruch 1, dadurch gekennzeichnet, daß sie durch Umsetzung mit kupplungsfähigen Diazoniumverbindungen bei pH-Werten zwischen 1 und 12 diazotiert worden sind.

5. Verfahren zur Herstellung von Derivaten des BPTI bei welchen ein Teil oder alle der Carboxylgruppen des nativen BPTI oder seiner durch Desamidierung erhaltenen Desamidoderivate modifiziert sind, dadurch gekennzeichnet, daß man BPTI oder

Le A 18 484

- 36 -

BPTI-Derivate mit Carbodiimiden im wäßrigen oder wasserhaltigen Medium bei pH-Werten zwischen 3 und 11 umsetzt, die entstandenen Reaktionsprodukte gegebenenfalls isoliert und/oder gegebenenfalls anschließend durch Umsetzung mit Nitrosylionen liefernden Agenzien oder mit nicht kuppelnden Diazoniumverbindungen desaminiert und/oder gegebenenfalls durch Umsetzung mit kupplungsfähigen Diazoniumverbindungen bei pH-Werten zwischen 1 und 12 diazotiert, und die jeweils entstehenden Reaktionsprodukte nach an sich bekannten Methoden isoliert.

6. Arzneimittel gekennzeichnet durch einen Gehalt an BPTI-Derivaten gemäß Anspruch 1.

7. Verwendung von BPTI-Derivaten gemäß Anspruch 1 bei der Bekämpfung von Krankheiten.

8. Verwendung von BPTI-Derivaten gemäß Anspruch 1 bei der Bekämpfung von Krankheiten, welche auf enzymatischen Disfunktionen beruhen.

BAD ORIGINAL

**Europäisches Patentamt**

# EUROPÄISCHER RECHERCHENBERICHT

0001773

Nummer der Anmeldung

EP 78 101 146.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | <u>DE - A - 2 619 246</u> (BAYER)<br>* Ansprüche 1, 4 * | 3, 4 |
| P | <u>DE - A - 2 654 124</u> (BAYER)<br>* Anspruch 13 * | 4 |
| D | Chemical Abstracts Band 67, Nr. 1, 1967<br>Columbus, Ohio, USA<br>D.G. HOARE et al "A method for the quantitative modification and estimation of carboxylic acid groups in proteins"<br>Seite 146, Spalte 2,<br>Abstract Nr. 144h<br><br>& J. Biol. Chem. Band 242, Nr. 10, 1967,<br>Seiten 2435 bis 2446 | 1, 2 |

./..

### KLASSIFIKATION DER ANMELDUNG (Int Cl.²)

C 07 G 7/00
A 61 K 37/64
C 07 G 7/02

### RECHERCHIERTE SACHGEBIETE (Int. Cl.²)

A 61 K 37/64
C 07 G 7/00
C 07 G 7/02

### KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| Berlin | 11-01-1979 | KNAACK |

| Kategorie | EINSCHLÄGIGE DOKUMENTE | | KLASSIFIKATION DER ANMELDUNG (Int.Cl.²) |
|---|---|---|---|
| | Kennzeichnung des Dokuments mit Angabe, soweit erforderlich, der maßgeblichen Teile | betrifft Anspruch | |
| D | Chemical Abstracts Band 70, Nr. 17 1969 Columbus, Chio, USA J. CHAUVET et al "Role of the carboxyl groups of pancreatic trypsin inhibitor (inhibitor of Kunitz) in its binding with trypsin" Seite 35, Spalte 2, Abstract Nr. 74 573 m & FEBS (Fed. Eur. Biochem. Soc.) Lett. Band 2, Nr. 1, 1968, Seiten 17 bis 19 - - - - | 1,2 | RECHERCHIERTE SACHGEBIETE (Int. Cl.²) |

BAD ORIGINAL